# EUROPEAN PATENT APPLICATION

(11) **EP 1 690 557 A1**
(43) Date of publication of application: **16.08.2006**
(21) Application number: 05002934.7
(22) Date of filing: 11.02.2005
(51) Int. Cl.: A61L 27/54, A61L 29/16, A61L 31/16, A61L 27/34, A61L 29/08, A61L 31/10, A61K 31/74, A61K 31/785

(54) **Device for treatment of rectal disorders, and manufacturing process for the same**

(71) Applicant: NOLabs AB, 252 21 Helsingborg (SE)
(72) Inventor: Peters, Tor, 8703 Erlenbach (CH)
(74) Representative: Karlsson, Leif Gunnar Börje

(57) **Abstract**

A device for therapeutic treatment of rectal disorders, including fissures, ulcers, haemorrhoids, and levator spasm, is disclosed. The device comprises a nitric oxide (NO) eluting polymer configured for eluting a therapeutic dosage of nitrogen oxide (NO) when used for the treatment. Furthermore, the device is configured for exposing a rectal treatment site of said rectal disorder in or on a rectal region of a body to said nitric oxide when said polymer elutes nitrogen oxide (NO). Moreover a method of manufacturing such a rectal treatment device is disclosed.

## Description

### Field of the Invention

This invention pertains in general to the field of treatment of rectal disorders, and in particular fissures, ulcers, haemorrhoids or levator spasm. More particularly the invention relates to a device for the therapeutic treatment of rectal disorders, and in particular fissures, ulcers, haemorrhoids or levator spasm, and a process for manufacturing of said device, involving the use of nitric oxide (NO).

### Background of the Invention

Rectal disorders, such as fissures, ulcers, haemorrhoids and levator spasm, are very common among adults in the world. Calculations estimate that over 50 percent of the population sometime experience these kinds of disorders. These disorders are often caused by the hectic way of life in the world of today, such as stress, straining, bad diet, and standing during long periods of time, or for instance after impacts on the rectal region when giving birth to a child.

Rectal fissures are small tears or cuts in the skin lining the anus. The symptoms of rectal fissures are blood in stool and pain during defecation. Rectal fissures may be acute, usually due to altered bowel habits, and chronic, deriving from bad bowel habits or an underlying medical problem. Today, the only treatment consists of keeping good hygiene, eating a healthy diet, and sometimes surgery.

Rectal ulcers may develop from many medical problems, such as ulcerative colitis, cancer, Crohn's disease, herpes, HIV etc. The common practice for treatment of rectal ulcers developed from medical problems, according to the background of the invention, is surgery. Needless to say, surgery is a method of treatment that causes great discomfort to the patient, and surgery is always a complicated procedure. In some cases medications are used, such as the hydrocortisone Proctofoam. These treatments are based on cortisone treatment. It is well documented that cortisone treatment does cause drug resistance against the cortisone. Moreover, the over exposure to cortisone has other adverse side effects on the human body.

Haemorrhoids are enlarged veins, just under the surface tissue, of rectum. Haemorrhoids that occur in rectum are called internal haemorrhoids, and those that occur around anus are called external haemorrhoids. The cause of haemorrhoids is not well documented, but causative suggestions are stress, straining, bad diet, and standing during long periods of time. Pregnant women often suffer from haemorrhoids. Up to this point haemorrhoids are treated with different kinds of creams, suppositories, ointments, containing different active substances, such as cortisone. These treatments are mainly based on cortisone treatment. Advanced treatments include sclerotherapy, which is an injection of a substance that shrinks the blood vessels, banding, which is an application of a tight elastic band around the haemorrhoid to cut of its blood supply, or surgical operation. These treatments are painful procedures. Moreover, these are treatments that need the assistance of a physician, and are therefore incompatible with self-treatment.

Levator spasm, or levator ani, is a functional anal disorder associated with great pain. The cause of the pain is a spasm in the muscles in the pelvic floor. This condition is very difficult to treat. The treatments according to the background of the invention include high voltage electro galvanic stimulation of the levator ani muscle, massage of the levator ani muscle, or medications, such as ibuprofen or naproxen. Treatment with high voltage electro galvanic stimulation is, needless to say, extremely painful, and requires equipment that the person suffering from levator spasm cannot keep at home. Therefore, this kind of treatment cannot give the patient an immediate relief of pain. Massage and medications do only offers moderate and momentary pain relief, and are not treatments giving a long lasting treatment of levator spasm.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide, or biocompatible without further affecting the patient before being removed from the body.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of treatment of rectal disorders, such as fissures, ulcers, haemorrhoids, and levator spasm, and the anti pathogenic potential of nitric oxide.

Hence, an improved device for the treatment and/or prevention of rectal disorders, which device does not develop resistance against the active pharmaceutical substance, is easy to apply, provides a painless treatment, has fast inset of treatment effect, and exclude the necessity of surgery, would be advantageous, and in particular a device allowing for target prevention and treatment of rectal disorders, such as fissures, ulcers, haemorrhoids, and levator spasm, would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device and manufacturing method according to the appended patent claims.

According to one aspect of the invention, a device is provided that allows for target treatment of rectal disorders, such as fissures, ulcers, haemorrhoids, and levator spasm. The device comprises a nitric oxide (NO) eluting polymer arranged to contact the area to be treated, such that a therapeutic dose of nitric oxide is eluted from said nitric oxide eluting polymer to said area.

According to another aspect of the invention, a manufacturing process for such a device is provided, wherein the process is a process for forming a device that allows for target treatment of rectal disorders, such as fissures, ulcers, haemorrhoids, and levator spasm. The process comprises selecting a plurality of nitric oxide eluting polymeric particles, such as nano fibres, fibres, nano particles, or microspeheres, and deploying said nitric oxide eluting particles in a condom/sheath or tape/coating to be comprised in said device.

The present invention has at least the advantage over the prior art that it does not develop resistance against the active pharmaceutical substance, is easy to apply, provides a painless treatment, has fast inset of treatment effect, and exclude the necessity of surgery target, by the exposure of an infected area to NO, whereby a very effective anti-fissure, anti-ulcer, anti-haemorrhoid and/or anti-levator spasm therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a condom/sheath according to the invention,

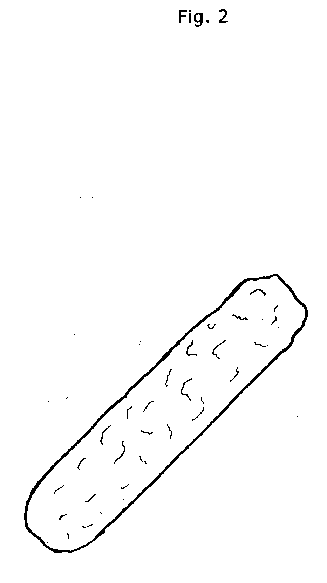

Fig. 2 is a schematic illustration of a condom shaped patch according to the invention,
Fig. 3 is a schematic illustration of a tampon according to an embodiment of the device of the invention, and
Fig. 4 is a schematic illustration of a rod comprised in an embodiment of the invention.

### Description of Embodiments

The following description focuses on embodiments of the present invention applicable to a device, for instance in form of a condom/sheath or tampon-like device, which allows for target treatment of rectal disorders, such as fissures, ulcers, haemorrhoids, and levator spasm.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide. Another advantage is that NO is released without any secondary products that could lead to undesired side effects.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

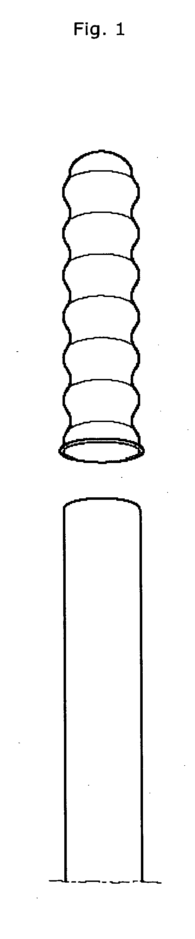

In an embodiment of the invention, according to Fig. 1, the device according to the present invention is in form of a latex or rubber condom/sheath, said condom/sheath being covered on the outside with nano-filament of NO-eluting L-PEI.

In another embodiment of the present invention the condom/sheath is covered on the outside with nano-filament of any other suitable polymer, according to above. Such polymers are for example polyalkyleneimines, such as B-PEI (Branched PolyEthyleneImine), which polymers have the advantage of being biocompatible, after the release of nitrogen oxide.

This condom/sheath may be in any suitable size, such as a suitable diameter size for inserting said condom/sheath in rectum. These diameter sizes may for example vary from small, medium, and large sized condoms/sheaths, such as 0.25 to 2 cm, for example 0.50 to 1 cm, such as 0.75. This condom/sheath may also vary in length, which length may be in the interval of 1 to 20 cm, such as 5 to 15 cm, for example 5, 6, 7, 8, 9, 10, 11, 12, 13, 14, and 15 cm.

The condom/sheath according to the present invention is applied by running said condom/sheath over a suitably sized stick, and then inserting said stick in rectum. When the stick is removed, the condom/sheath left in rectum. As soon as the condom/sheath according to the present invention gets in contact with the moisture in rectum, the condom/sheath starts to elute NO on the area to be treated. When treatment is finished or to be interrupted, the device is easily retracted from the rectal region for that purpose.

In another embodiment of the present invention the condom/sheath according to the present invention is covered on the outside with NO-eluting nano-particles, or micro-spheres. These nano-particles, or micro-spheres, may be formed from the NO-eluting polymers according to the present invention, encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable or biocompatible polymers, and other soluble plastics. When the nano-particles, or micro-spheres, according to this embodiment, gets in contact with the moisture in rectum on the outside of the condom/sheath, they start to elute NO on the area to be treated.

In still another embodiment the device according to the present invention may be manufactured in the form of a rod shaped patch, made of NO-eluting polymer according to the present invention. This rod-shaped patch is flexible, which results in that the person to be treated, in a small extension, feels the rod shaped patch during treatment.

In yet another.embodiment said rod shaped patch is manufactured of a basic material, which basic material is covered with the nano-fibres, nano-particles, and/or micro-spheres according to the invention. The basic material of this rod, according to the invention, may be polyvinylacetates, polylacticacids, starch, cellulose, polyhydroxyalkanoates, polyesters, polycaprolactone, polyvinylalcohol, polypropylene, polycarbonates, polyurethanes, polyethers, polyamides, polyacrylonitriles, polystyrenes, protein based plastics, gelatine, and other biocompatible polymers.

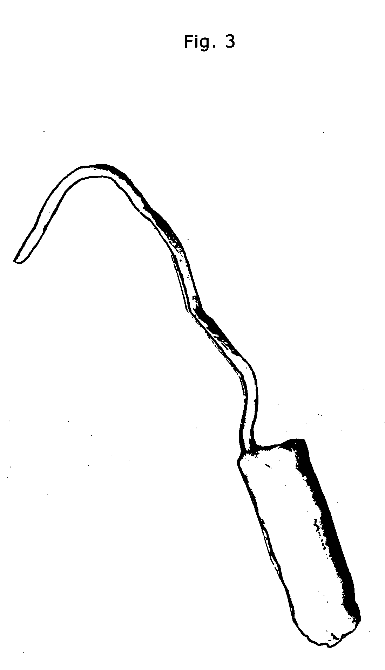

In another embodiment the device according to the invention is in form of a tampon-like shape, according to Fig. 3, covered on the outside of NO-eluting nano-fibres, nano-particles, or micro-spheres according to the present invention. The basic material of this tampon-like device may be any of the NO-eluting polymers according to the invention, or polyvinylacetates, polylacticacids, starch, cellulose, polyhydroxyalkanoates, polyesters, polycaprolactone, polyvinylalcohol, polypropylene, polycarbonates, polyurethanes, polyethers, polyamides, polyacrylonitriles, polystyrenes, protein based plastics, gelatine, and other biocompatible polymers.

The tampon according to the present invention may also be manufactured of other materials, including normal tampon materials, such as cotton. In this case the NO-eluting polymer, such as L-PEI, may be spun onto said tampon. The size of said tampon may vary, but in one embodiment of the present invention the size corresponds to the smallest tampon size, such as a tampon with a diameter to 1.5 cm, for example 0.50 to 1 cm, such as 0.75 cm. The tampon according to the invention may optionally be equipped with a retracting string, for easier retraction of the tampon from rectum.

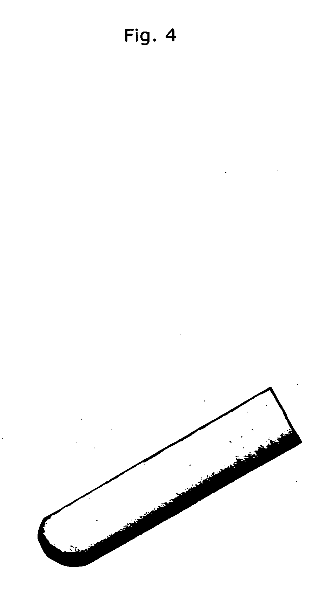

In still another embodiment of the present invention the device is a rod, according to Fig. 4, manufactured of NO-eluting polymer, according to the present invention.

In another embodiment said rod is manufactured as a carrier and of a basic material, which basic material is covered with the nano-fibres, nano-particles, and/or micro-spheres of NO-eluting polymer according to the invention. The basic material of this rod, according to the invention, may be polyesters, polyamides, polyethers, polyurethanes, polycarbonates, polyvinylacetates, polylacticacids, starch, cellulose, polyhydroxyalkanoates, polyesters, polycaprolactone, polyvinylalcohol, protein based plastics, gelatine, and other biocompatible polymers. The NO-eluting polymer may be integrated in, or spun together with, any of these materials.

The diameter of the rod according to the invention may vary, but in one embodiment of the present invention the diameter may be in the interval 0.25 to 1.5 cm, for example 0.50 to 1 cm, such as 0.75 cm.

The rod shaped patch, the tampon, or the rod is inserted in rectum. As soon as the rod shaped patch, the tampon, or the rod according to the present invention gets in contact with the moisture in rectum, the rod shaped patch, the tampon, or the rod starts to elute NO on the area to be treated.

In another embodiment these nano-particles, or micro-spheres, may be integrated in a soluble film that disintegrates on the outside of the condom/sheath, condom shaped patch or rod according to the present invention, in order to elute NO at the area of interest when the soluble film gets in contact with the moisture in rectum, on the area to be treated.

When the NO-eluting device, according to the embodiments of the present invention, gets in contact with the moisture in rectum, said device starts to release NO to the area to be treated. This device does not develop resistance against nitric oxide (NO), is easy to apply, provides a painless treatment, has fast inset of treatment effect, and exclude the necessity of surgery target.

When placed on an area to be treated the device according to the present invention provides prevention and treatment of rectal disorders, such as fissures, ulcers, haemorrhoids, or levator spasm.

In yet another embodiment of the present invention the NO-eluting device is acting as a booster for drug eluting patches, e.g. pharmaceuticals, vitamins, nicotin, nitroglycerin etc. This embodiment presents a device with the advantage of combining two therapeutic treatments, of significant value, in one treatment. Hence, a synergetic effect may be achieved by such devices when NO that is eluted from the device. NO has a vasodilatory effect on the region where the device having the combination compound actuates. Vasodilated tissue is more susceptible to certain medications and thus more easily treated by the medical preparations and still NO has in addition to that the anti-inflamatory, anti-bacterial etc. effect. Hence, an unexpected surprisingly effective treatment is provided.

In another embodiment of the device according to the present invention the fibres, nano-particles, or micro-spheres may be integrated in a gel, cream, or foam that may either be in a smearing or compressed structure. The elution of NO may then be initiated by inserting said gel in rectum. The fibres, nano-particles, or micro-spheres may also be integrated in a hydrogel, which is mixed directly before use. This embodiment has the advantage of being able to penetrate pockets and corners in rectum, or in the anal area, for closer elution of NO on the area to be treated. A gel, foam, ointment etc. may also be combined with the above mentioned drug boosting effect.

The device according to the present invention elutes nitric oxide (NO) from said eluting polymer in a therapeutic dose in the oral cavity, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

The devices according to the embodiments of the present invention may be in any suitable diameter sizes, such as a suitable diameter size for inserting said device in rectum, according to above. These devices may also vary in length, which length may be in the interval of 1 to 20 cm, such as 5 to 15 cm, for example 5, 6, 7, 8, 9, 10, 11, 12, 13, 14, and 15 cm.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The basic material of the device according to the present invention may be polyesters, polyamides, polyethers, polyurethanes, polycarbonates, polyvinylacetates, polylacticacids, starch, cellulose, polyhydroxyalkanoates, cotton, polyesters, polycaprolactone, polyvinylalcohol, polyacrylonitrile, polystyrene, poly(acrylic acid), polypropylene, protein based plastics, gelatine, and other biocompatible polymers. The NO-eluting polymer may be integrated in, spun together with, or spun on top of, any of these materials in all of the embodiments of the present invention.

The device according to the present invention may be manufactured by, for example electro spinning of L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the certain embodiments of the device according to the invention. L-PEI may also be combined with other polymers comprising L-PEI or being arranged in combination with L-PEI.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres with the area to be treated, effective use of NO-eluting polymer, and a cost effective way of producing the device according to the present invention.

Hereinafter, some potential uses of the present invention are described:

A method of therapeutical treatment of rectal disorders, including fissures, ulcers, haemorrhoids, and levator spasm by means of a device comprises a nitric oxide (NO) eluting polymer configured for eluting a therapeutic dosage of nitrogen oxide (NO) when used for said treatment, comprising exposing a rectal treatment site of said rectal disorder in or on a rectal region of a body to said nitric oxide when said polymer in use elutes nitrogen oxide (NO) by eluting a therapeutic dose of nitric oxide from said nitric oxide eluting polymer to said treatment site.

The method according to the above, wherein said rectal treatment site of said rectal disorder is a rectum, and wherein said method comprises applying a condom/sheath, a rod shaped pad, rod, and/or a tampon to said rectum for said exposure.

Use of nitric oxide (NO) in a therapeutic dose for therapeutically treating rectal fissures, ulcers, haemorrhoids, and/or levator spasm.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first ", "second" etc do not preclude a plurality. Reference signs in the claims are provided merely as a clarifying example and shall not be construed as limiting the scope of the claims in any way.

## Claims

**1.** A device configured for therapeutic treatment of rectal disorders, including fissures, ulcers, haemorrhoids, and levator spasm, wherein
said device comprises a nitric oxide (NO) eluting polymer configured for eluting a therapeutic dosage of nitrogen oxide (NO) when used for said treatment, and
wherein said device is configured for exposing a rectal treatment site of said rectal disorder in or on a rectal region of a body to said nitric oxide when said polymer in use elutes nitrogen oxide (NO).

**2.** Device according to claim 1, wherein said polymer is selected from the group consisting of polyalkyleneimines, S-nitrosylated polymer, and poly(alkylenimine)diazeniumdiolates, or any combination thereof.

**3.** Device according to claim 1, wherein said polymer is L-PEI (linear polyethyleneimine), loaded with nitric oxide (NO), arranged for release of the nitric oxide (NO) at said rectal treatment site of said rectal disorder.

**4.** Device according to claim 1, in a form selected from the group comprising of a condom/sheath, a rod shaped pad, rod, and a tampon, adapted to be applied on or at said rectal treatment site.

**5.** Device according to claim 4, wherein said condom/sheath, condom shaped pad, rod, or tampon is a polyurethane, latex, rubber, or polyethylene condom/sheath, condom shaped pad, rod, or tampon, including said nitric oxide (NO) eluting polymer configured for in use eluting said nitric oxide (NO) to said rectal treatment site said treatment thereof.

**6.** Device according to claim 1, wherein said device is partly disintegrable when subjected to moisture or water.

**7.** Device according to claim 1, wherein said polymer comprises silver, configured for therapeutic treatment of said rectal treatment site of said rectal disorder.

**8.** Device according to claim 1, configured to act as a booster for drugs or pharmaceutical compounds.

**9.** Device according to claim 1, wherein said polymer is comprised in said device in form of nano-particles or micro-spheres.

**10.** Device according to claim 9, wherein said nano-particles or micro-spheres are integrated in a gel, hydrogel, foam or cream.

**11.** Device according to claim 9, wherein said nano-particles, or micro-spheres, are encapsulated in a material, selected from the group consisting of polyvinylacetates, polylacticacids, starch, cellulose, polyhydroxyalkanoates, polyesters, polycaprolactone, polyvinylalcohol, protein based plastics, and gelatine, and combinations thereof.

**12.** Device according to claim 1, wherein said device comprises a material which regulates or controls NO-elution, selected from the group consisting of as polyvinylacetates, polylacticacids, starch, cellulose, polyhydroxyalkanoates, polyesters, polycaprolactone, polyvinylalcohol, protein based plastics, gelatine, and/or biogradable polymers.

**13.** Device according to claim 1, wherein said NO-eluting polymer is applied on, or integrated with, a material selected from the group consisting of polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, and gelatine, or combinations thereof.

**14.** A device according to any of the preceding claims, wherein said nitric oxide (NO) eluting polymer is activateable to elute said therapeutic dosage of nitrogen oxide (NO) by water.

**15.** A manufacturing process for a device according to claim 1, comprising:
selecting a plurality of nitric oxide eluting polymeric particles, preferably nanoprticles or fibres,, and
deploying said nitric oxide eluting particles into a suitable form, said deploying comprises electro, air, or gas stream spinning of said fibres.

**16.** Use of a nitric oxide (NO) eluting polymer for the manufacture of a device for the treatment of rectal disorders, including fissures, ulcers, haemorrhoids, and levator spasm wherein
said nitric oxide is loaded to said device elutes nitric oxide (NO) from said eluting polymer in a therapeutic dose when used at a site of infection in or on a body.

**16.** Use according to claim 15, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
